# EUROPEAN PATENT SPECIFICATION

(11) **EP 4 373 532 B1**
(45) Date of publication and mention of the grant of the patent: **01.07.2026**
(21) Application number: 22754089.5
(22) Date of filing: 22.07.2022
(51) Int. Cl.: A61L 24/00, A61L 24/10, A61L 26/00, A61L 31/04, A61L 31/14

(54) **BIO-ADHESIVE**
BIOKLEBSTOFF
BIO-ADHÉSIF

(30) Priority: 23.07.2021 NL 2028827
(43) Date of publication of application: 29.05.2024
(73) Proprietor: SentryX B.V., 3584 CM Utrecht (NL)
(72) Inventor: PILUSO, Susanna, 3711 AA AUSTERLITZ (NL); VAN TOL, Floris Rudolf, 3711 AA AUSTERLITZ (NL); STEVERINK, Jasper Gerard, 3711 AA AUSTERLITZ (NL); DE VRIES, Anne-Mare Sofie, 3711 AA AUSTERLITZ (NL)
(74) Representative: V.O.
(86) International application number: PCT/EP2022/070622
(87) International publication number: WO 2023/002017

(56) References cited:
- WO-A1-2021/250205
- US-A1- 2020 199 199
- US-A1- 2020 299 556
- GOWDA ADARSHA H J ET AL: "Design of tunable gelatin-dopamine based bioadhesives", INTERNATIONAL JOURNAL OF BIOLOGICAL MACROMOLECULES, ELSEVIER BV, NL, vol. 164, 25 July 2020 (2020-07-25), pages 1384 - 1391, XP086335588, ISSN: 0141-8130, [retrieved on 20200725], DOI: 10.1016/J.IJBIOMAC.2020.07.195

## Description

### Technical Field

This invention concerns a bio-adhesive.

### Background Art

Mussel adhesive proteins (MAPs) have been suggested as potential alternative for tissue adhesives. These adhesive proteins, secreted by marine mussels, enable them to adhere to a wide range of underwater surfaces. Although the exact chemical adhesion mechanism is still unknown, MAPs are considered one of the most powerful adhesives, even stronger than other polymer-based adhesives such as, epoxy and phenolic resins.

From WO2021059272 it is known that L-3,4-dihydroxyphenylalanine (L-DOPA) and its derivatives, such as 3,4-dihydroxyhydrocinnamic acid or 3-(3,4-dihydroxyphenyl)propionic acid, (commonly referred to as dihydrocaffeic acid or DHC) have been chemically coupled with synthetic and natural polymers to improve the efficacy of bio-adhesives presently used. Murphy et al. (2010), for example, presented a bio-adhesive based on the covalent bonding of DHC to polycaprolactone (PCL) and poly(ethylene glycol) (PEG). Furthermore, it was reported that the incorporation of nano-particle sized LAPONITE^{®} enhanced the mechanical properties of a similar tissue adhesive due to strong conjugation between the LAPONITE^{®} and the catechol moieties of dopamine (Liu et.al., 2014). PEG was also used to increase the hydrophilicity of a PEG/silk fibroin adhesive hydrogel. The silk fibroin was chemically coupled with dopamine. The hydrogel showed improved water solubility and adhesion strength compared to the silk without catechol functionalization (Burke, et.al., 2015). It was also reported that the polar functional group of the catechol can be utilized to improve both chitosan solubility and bio-adhesive properties (Ryu, etal., 2015). Fan et al. (2016) reported an aldehyde-free tissue adhesive by conjugating dopamine to a gelatin macromer intended for internal medical use. The adhesive was cross-linked by two cross-linking systems enhancing a rapid formation of wet adhesion and long-term resistance. Another research team developed a tissue adhesive for sternal bone closure based on the chemical modification of hyperbranched poly(β-amino ester) with poly(dopamine-co-acrylate). The adhesive has been reinforced with nanosized hydroxyapatite particles. The adhesion mechanism combined both catechol moieties from the dopamine and the cross linkable vinyl functional groups leading to high mechanical strength, high wet adhesion properties and tailored curing speed by using different cross-linkers. Zhang et al. (2014) also co-polymerized dopamine with hyperbranched poly (β-amino ester) and poly(dopamine-co-acrylate).

WO2021059272 itself discloses a catechol-base hot-melt tissue adhesive for hernia mesh repair surgery that is based on a bio-adhesive composition comprising at least one caffeic acid derivative and at least one synthetic thermoplastic polymer, wherein exposure to heat causes the bio-adhesive composition to transform into a non-solid state and to cohesively adhere to a biological tissue upon subsequent cooling thereof.

Different from what is suggested in WO2021059272, the chemical coupling of catechol moieties to a polymer is not at all that difficult. However, it remains of interest to prepare polymers with mixed catechol and quinone functionality, which have improved adhesiveness to a wide variety of substrates.

From WO2016010484A1 a biological tissue adhesive composition is known, as well as a method of preparation thereof. The biological tissue adhesive composition comprises one or more macromolecules grafted with at least one catechol moiety (e.g., selected from the group consisting of dopamine, dihydroxyphenylalanine, DHC and combinations thereof) and comprising at least one cross-linkable functional group. For example, a solid mixture of a gelatin-dopamine conjugate (or other gluing macromer) and genipin (or other long-term acting crosslinker) may be dissolved in normal saline solution; and be smeared on tissue surface with a cotton bud, followed by the drip of a multivalent metal ion solution (acting as fast cross-linking agent) with a syringe on the tissue surface (coated with mixture solution of gelatin-dopamine conjugates and genipin), and then tissue surfaces may be adhered after gentle press for about 10 seconds. The gelatin-dopamine conjugate is made by EDC/NHS coupling chemistry.

WO2015017562A1 discloses adhesive materials comprising polymers with catecholic functionality as adhesives, and methods of making and using the same. From this reference it is known that unprotected catechols can irreversibly crosslink in air at neutral or basic pH, which can limit the shelf life of such materials. Cohesive interactions between polymer films functionalized with exposed and blocked catechols are mentioned. Moreover, catechol surfaces were partially or fully oxidized to quinone surfaces by adding different concentrations of periodate.

In the Journal of Materials Chemistry B, 10.1039/C4TB01196A, a paper is provided on "In vitro and in vivo assessments of 3-(3,4-Dihydroxyphenyl)-2-propenoic acid bioconjugated gelatin based injectable hydrogel for biomedical applications" by Selvaraj Thirupathi Kumara Raja et al. In this study, an attempt was made to construct an injectable hydrogel through bioconjugation of dihydroxy phenolic acids to the gelatin backbone. Bioconjugating caffeic acid with gelatin followed by oxidation with mild oxidation agents provide a hydrogel with all the requisite properties (biocompatibility, controlled biodegradability, antioxidant, antimicrobial and wound healing). Bioconjugation was performed using EDC/NHS and the resultant gel named as Caffeic acid Bioconjugated Gel (CBG gel). CBG gel promotes cell migration and demonstrates radical scavenging behavior. When subcutaneously injected into the animal, as *in situ* CBG gel, the gel was highly biocompatible and does not influence any necrosis and the cross talk with the adjacent tissue cells was smooth and the gel completely degraded within 24 days. The wound healing efficacy on full thickness wound suggested that CBG gel accelerate healing and impart high strength to the healed skin at appreciable level. With all these added functional properties, CBG gel finds biomedical applications.

US2020299556 relates to a coacervate formed from a catechol-substituted anionic polymer; an adhesive comprising same; and a method for producing same, by mixing a catechol derivative of a mussel adhesive protein and a catechol-substituted anionic polymer.

US2020199199 relates to a multiblock copolypeptide having stimulus responsivity and surface adhesiveness. The multiblock copolypeptide of the present disclosure, which is composed of an elastin-based polypeptide and a mussel foot protein, can form self-assembled core-shell structures and hydrogels exhibiting reversible change in response to temperature stimulation and can be used for biomedical applications.

In "Design of tunable gelatin-dopamine based bioadhesives", Gowda et al, Int J Biol Macromol.. 2020 Dec 1;164:1384-1391, a study is provided wherein the authors have fabricated a series of gelatin-dopamine (Gel-dop) conjugates and studied their adhesive properties after being chemically crosslinked using sodium periodate. This study illustrates that adhesiveness can be regulated by changing the degree of dopamine substitution.

In co-pending application WO2021250205 an adhesive drug carrier is disclosed comprising an injectable hydrogel comprising at least one medication, wherein the hydrogel comprises a (i) a protein-based polymer functionalized with a functionalization agent that is able to form guest-host interactions with oxidized β-cyclodextrin, cross-linked with (ii) an oxidized β-cyclodextrin (oβ-CD) as matrix and the at least one medication (iii), and wherein the hydrogel further comprises (iv) a protein-based polymer bearing quinone and/or catechol groups. In this case, the protein-based polymer (iv) bearing quinone and/or catechol groups is but a minor component included in the matrix based on oβ-CD.

The prior art includes references disclosing catechol and the quinone moiety as essential components in achieving bulk adhesivity. However, despite the extensive research inspired by mussel adhesive proteins, it remains desirable to have a bio-adhesive that is highly biocompatible and has excellent cross-material adhesion to tissue, bone, metal as well as medical hydrogels, for instance, that allows tissue-bone, or bone-metal, or metal-hydrogel adhesion, etc. In other words, it remains highly desirable to achieve a desired ratio of catechol and quinone functionality in a controlled and elegant manner.

### Summary of the Invention

The present invention is defined by the claims and provides a bio-adhesive comprising one or more protein-based polymers with catechol and quinone functionality wherein the polymer is substituted with at least 2 different functionalization agents each having a 3,4-dihydroxyphenyl group and wherein the at least 2 different functionalization agents are oxidized to a different degree.

More in particular, the present invention provides a bio-adhesive comprising one or more protein-based polymers with catechol and quinone functionality wherein the polymer is substituted with at least 3-(3,4-dihydroxyphenyl)propanoic acid (dihydrocaffeic acid, DHC) and 3-(3,4-dihydroxyphenyl)propenoic acid (caffeic acid, CA), wherein the DHC and CA are oxidized to a different degree, wherein
- the protein-based polymer is selected from silk, fibrin, collagen and/or gelatin, and
- the ratio of the functionalization agents and amount of oxidant are selected such as to achieve a conversion of 4 to 96% of the catechol moieties into quinone moieties.

The protein-based polymer is selected from silk, fibrin, collagen and/or gelatin, and makes up at least 50% by weight on the entire polymer content of the bio-adhesive.

The present invention further provides a bio-adhesive for application on the outside or inside of a living human or animal body. Suitably, this may be in the form of a kit of parts. For instance, a dual barrel syringe may be used, connected to a mixing tip.

### Drawings

Fig. 1, a schematic representation of the bio-adhesive of the present invention in a total hip replacement.
Fig. 2, a schematic representation of application in a wound closure procedure.
Fig. 3, a schematic representation of an intestinal anastomosis, where two hollow structures are glued together.
Fig. 4, a schematic representation of the bio-adhesive of the present invention which is used to adhere bone fragments after fracture healing.
Fig. 5 (a and b), a schematic representation of the bio-adhesive of the present invention which is used to adhere a hydrogel to bone or a surgical plate.

The above figures are for illustration purposes only and not necessarily to scale.

### Detailed description of the invention

The present invention focusses on bio-adhesives that are based on protein-based polymers. These polymers are biocompatible and can be applied and used outside a body, but more importantly also *in vivo.* Moreover, they are biodegradable, i.e. can be decomposed naturally inside the human body. Accordingly, the protein-based polymer is a commercially available biocompatible polymer that comprises amino and carboxylic groups, selected from silk, fibrin, collagen or gelatin. More preferably, the bio-adhesive used in the present invention is based on gelatin. The bio-adhesive may also comprise other biocompatible water-soluble synthetic or natural polymers. The other polymers may compose up to 50% by weight on the entire polymer content. In other words, the protein-based polymer selected from silk, fibrin, collagen or gelatin, makes up at least 50% by weight on the entire polymer content of the bio-adhesive. Given its availability, biocompatibility and cost, the use of gelatin as sole polymer component is preferred.

The protein-based polymer is or the polymers are reacted with at least 2 different reactants bearing a catechol group or a derivative thereof. In the present invention said different reactants are 3-(3,4-dihydroxyphenyl)propanoic acid (dihydrocaffeic acid, DHC) and 3-(3,4-dihydroxyphenyl)propenoic acid (caffeic acid, CA). For instance, the protein-based polymer may be modified with two or more catechol-containing compounds or two or more protein-based polymers may each be modified with a different catechol-containing compound. Furthermore, the reactants can preferably be easily oxidized to provide quinone-groups.

Also disclosed are reactants such as 2-(3,4-dihydroxyphenyl)ethylamine hydrochloride (dopamine), 3,4-dihydroxy-L-phenylalanine (L-DOPA), 3,4,5-trihydroxybenzoic acid (gallic acid), (*R*)-4-(1-hydroxy-2-(methylamino)ethyl)-1,2-benzenediol (epinephrine), or (*R*)-4-(2-amino-1-hydroxyethyl)-1,2-benzeendiol (norepinephrine). In accordance with the present invention, the catechol-containing compounds are partly oxidized into quinone-groups, thereby creating dual functionality. This dual functionality achieves adhesion to e.g., soft tissue (quinone) together with adhesion to metals and other materials used in surgical implants (catechol). Derivatives of e.g., CA or DHC, with the functional group being temporarily protected, may also be used. Of importance is the biocompatibility of the reactants to avoid any toxicity issues. Given its availability, biocompatibility and cost, the use of 2 or more reactants selected CA, DHC or the protected derivatives thereof is preferred. More preferably 2 reactants are selected with a different oxidation rate. For instance, a combination of reactants may be used allowing 50% conversion to quinone of the faster oxidizing catechol moiety, whilst less than 25%, preferably less than 10%, preferably less than 5% of the slower catechol moiety is converted into quinone. Using at least 2 different reactants with significantly different oxidation rate hence allows great control of maintaining catechol groups and creating quinone groups, in a single oxidation step, thus creating adhesion to a combination of substrates. Most preferably, a combination is used of DHC and CA, wherein the CA in a subsequent step is oxidized, with little or no oxidation of the DHC. The faster oxidizing functionalization agent and the slower oxidizing agent may be used at various molar ratios. For instance, they may be used at a molar ratio in the range of 10:1 to 1:10, preferably at a molar ratio in the range of 5:1 to 1:5.

In order to achieve sufficient adhesion, preferably between 15 to 70% more preferably between 20 to 50% of the combined carboxylic acid and amino groups of the protein-based polymer(s) are functionalized to bear a catechol or (upon oxidation) quinone moiety. If the degree of functionalization is below 15 % of the combined carboxylic acid and amino groups, then the adhesive performance may be too low. When reacting more than 70% of the combined carboxylic acid and amino groups, achieving a homogeneous bio-adhesive may become problematic. Moreover, the remaining carboxylic acid and amino groups contribute to the cohesion by reacting with quinone moieties. Removing more than 70% thereof therefore impacts the cohesion. The interaction between the aromatic ring of catechol and positively charged residues, such as lysine (cation-π interaction), has been recognized as an important non-covalent interaction in mussel adhesion. (Ref Nanoscale, 2020,12,1307-1324; Angewandte Chemie International Edition, 2019, 58.3: 696-714.) Preferably the amino groups rather than the carboxylic acid groups of the protein-based polymer(s) are functionalized.

The protein-based polymer preferably has a molecular weight in the range of 20 to 250 kDa, preferably 50 to 200 kDa, more preferably 90 to 150 kDa. Below 20 kDa the cohesion of the bio-adhesive may be insufficient. Above 250 kDa achieving a homogeneous bio-adhesive may become problematic.

The protein-based polymer(s) bearing different catechol moieties may be oxidized with any suitable oxidant. For instance, chemical oxidants such as sodium periodate, sodium persulfate, hydrogen peroxide, or enzymatic oxidants such as horseradish peroxidase, tyrosinase may be used. The ratio of the functionalization agents and amount of oxidant are selected such as to achieve a conversion of 10 to 90%, preferably 20 to 80%, more preferably 30 to 70% of the catechol moieties into quinone moieties. If a high quinone to catechol ratio is desired, then preferably a high ratio of faster oxidizing functionalizing agent to slower oxidizing functionalizing agent is used. For tissue to tissue adhesion, the molar ratio between catechol moieties and quinone moieties is preferably in the range from 1:5 to 1:24, more preferably from 1:7 to 1:20, more preferably about 1:10. For tissue to metal adhesion, the molar ratio between catechol moieties and quinone moieties is preferably in the range from 7:1 to 1:7, more preferably from 5:1 to 1:5, more preferably about 1:1. For metal to metal adhesion, the molar ratio between catechol moieties and quinone moieties is preferably in the range from 5:1 to 24:1, more preferably from 7:1 to 20:1, more preferably about 10:1.

In a co-pending application, the use of a hydrogel as anaesthetic-carrying body for local release of medication in the form of a ring is described (WO2019117715) where it is used in combination with a screw. In an alternative embodiment, the anaesthetic-carrying body is glued on a plate element. Information on a suitable manner to glue the anaesthetic-carrying body onto a plate element is not provided. By applying the bio-adhesive of the present invention, the need for a screw or similar form of attachment is avoided.

The bio-adhesive may be applied on the outside or inside of a living human or animal body. For instance, it may be applied to lacerations of the skin on the outside, but also to 'wound' closures inside the body, e.g., creating surgical anastomoses of vascular structures or intestines, or other hollow structures like the bile duct. Additional examples where a glue could be useful are the closure of dural tears during surgery, or 'sticking' the visceral pleura to the parietal pleura of the lung during a video-assisted thoracoscopy, or injection into a herniated / degenerated intervertebral disc. This would avoid the current use of chemical irritants.

The bio-adhesive may be applied as mixture of components in a viscous form. In this manner, it can be applied onto irregular or tight anatomical spaces, without the need for a predefined form. It may be used to adhere soft tissue to soft tissue. For instance, it may be applied as a topical skin adhesive where it reacts spontaneously upon contact with weak bases such as water, blood or cell membranes, to assist in wound closure. It may also be used to adhere soft tissues inside the body to repair for instance tissue lacerations, for instance, blood vessels or bowel. It may similarly be used to attach soft tissue to surgical implants. For instance, it may attach tendons to a surgical plate after fracture reduction, e.g., where such tendons are either traumatically/degenerately torn or had to be surgically released during exposure of the fracture. Moreover, the adhesive drug carrier may be used to aid in fixation of fractures, whereby bone fragments are attached to each other or to other surgical elements. Components advancing the healing process or strengthening the juncture formed by the bio-adhesive may be added. Moreover, it may be used to attach soft tissue and/or bone to metal. Existing hydrogels may be attached in a similar manner.

Each of these embodiments is very suitable for treatment of medical disorders such as musculoskeletal disorders, and especially for treatment of skeletal disorders due to the ability of the bio-adhesive to adapt to the shape of a bone, tissue or surgical implant to which it has been applied. This ability is of interest also in other applications where its presence if it would lack flexibility would cause hindrance to the patient and/or restrict movement. These disorders include infection, inflammation, auto-immune disease, malignant and benign neoplasms, growth disorders, trauma, degenerative disorders or treatment of pain arising from (surgical treatment of) these disorders.

The bio-adhesive of the present invention may contain medication, such as an anaesthetic. The medication may also be or comprise an antibiotic or anticancer agent, a growth factor, an immunomodulatory drug, chemotherapeutic agents, steroids (including retinoids), hormones, anti-microbials, antivirals, anti-inflammatory compounds, radiation absorbers, including UV-absorbers, radiation enhancers, a hemostatic agent, vaccines, stem cells, etc. The medication may furthermore be hydrophilic or hydrophobic.

The bio-adhesive may comprise additional components, like colorants, stabilizers, preservatives, antioxidants, co-solvents, buffers and similar common additives.

Further ingredients may be included, preferably further ingredients selected from co-medication, co-solvents, surfactants, colorants, and buffers. Co-medication may be considered to be any further medication added to the bio-adhesive, preferably medication which advances the healing process. Co-solvents include but are not limited to plasticizers. One such plasticizer is glycerol. The addition of glycerol into the bio-adhesive results in higher elasticity, yet does not affect sample stiffness. Other co-solvents may be chosen for their ability to improve drug solubility during loading, such as DMSO or ethanol. Substances that can change the degree or rate of oxidation of the catechol moieties into quinone moieties may also be added.

Methods for adding catechol-functionality to protein-based polymers are known. Thus, it is known to functionalize gelatin and related protein-based polymers with EDC/NHS through activation of carboxylic groups and reaction with amino groups. Alternatively, catechol-functionality can be introduced via NHS-esters of catechol molecules. Biomaterials, 31 (2010), pp. 1148-1157 describes the functionalization of gelatin using EDC/NHS. Biomaterials 31 (2010) 8323e8331 describes the functionalization of gelatin using the Bolton-Hunter reagent, which is an NHS ester. Of importance, but common in the field of medical application, is to remove all forms of contamination. By way of example, the bio-adhesive may be prepared by the following method:
1. A solution of a protein-based polymer bearing a fast oxidizing catechol moiety is mixed with a solution of a protein-based polymer bearing a slow oxidizing catechol moiety.
2. Optional additives are added.
3. These solutions, are mixed so that pre-determined concentrations are obtained. These concentrations can be varied depending on the desired mechanical and release properties.
4. An oxidizing agent is added to the obtained solution to create dual functionality. Whereafter the bio-adhesive is applied onto at least one of the surfaces that need to be attached.
5. Adhesion is achieved after pressing the surfaces together.
6. Alternatively, a solution is provided of a protein-based polymer bearing pre-determined amounts of fast and slow oxidizing catechol moieties.
7. Optional additives are added.
8. An oxidizing agent is added to the obtained solution to create dual functionality. Whereafter the bio-adhesive is applied onto at least one of the surfaces that need to be attached.

The bio-adhesive may be applied with a device that mixes the components upon use. For instance, a dual barrel syringe may be used, connected to a mixing tip. In this case the bio-adhesive would be used in the form of a kit of components. For instance, one syringe would contain the protein-based polymer(s) and the other the remaining components.

In this case, the bio-adhesive can be injected directly from the mixing tip to a tissue or metal plate.

By way of example, (components of) the bio-adhesive may be prepared as follows:

### Recipe 1 (catechol or quinone functionality):

In a first step gelatin with a MW of 120 kDa was functionalized with DHC or CA in an amount of 2.5, 5 or 10%wt respectively. Various routes are available:
- example of functionalization with CA: EDC/NHS is used to activate the carboxylic group of CA and then reacted with the gelatin lysine amino groups at pH of about 4.5-5.5. The gelatin-CA (GCA) conjugate is then purified in an acidic environment (e.g. dialysis against HCl solution) to prevent the oxidation to quinone.
- example of functionalization with DHC: DHC is activated with EDC/NHS in MES buffer (pH 4.5-5.5) and then reacted with gelatin lysine amino groups. Purification of the gelatin-DHC conjugate (GDHC) is similar to previous.

Alternatively, NHS-esters of DHC and CA can be prepared and then reacted with the amino groups of gelatin.

For the synthesis of NHS-ester of DHC (or NHS ester of CA), a solution of DHC in dimethylformamide (DMF) is reacted with polymer-bound HOBt using PyBOP (Benzotriazol-1-yloxy) tripyrrolidinophosphonium hexafluorophosphate) as coupling agent, at room temperature in the presence of an organic base (e.g. di-isopropylethylamine or pyridine). The synthesis is performed in a filtered column for peptide synthesis. The polymer is then filtered, washed with chloroform, DCM and dried with diethylether. The dried polymer-DHC is then suspended in DCM, followed by NHS and reacted for 4-8h. After reaction, the mixture is filtered and washed with DCM. Filtrate and washing are then combined and the product is collected by evaporating the solvent under reduced pressure.

The NHS ester of DHC and/or CA is reacted with gelatin dissolved in a buffer(pH between 6-9.0) at 45 °C, for 2h. The reaction mixture is then purified by dialysis, or precipitation in cold ethanol.

For quinone functionality, the GCA or GDHC is dissolved in an alkaline solution (e.g. pH 8-8.5) to promote the oxidation of the catechol groups to quinone. The catechol moieties in GCA, as indicated above, oxidize much more readily than those of GDHC to quinone. Quinone may also be formed by the addition of an oxidizing agent, e.g., SPS or H₂O₂.

### Recipe 2 (quinone and catechol functionality):

The double functionality may for instance be obtained by mixing GCA and GDHC, followed by oxidation. When using mild conditions, the formation of quinones will only occur for GCA, bearing the fast oxidizing catechol moieties.

### Example 1, adhesion test

Gelatin was functionalized with either DHC or CA to obtain functionalized gelatin: GDHC and GCA, respectively. The degree of functionalization (amount of free NH₂ groups which are now attached to DHC or CA) was similar for both gelatin batches (42.4 and 40.3 %). To prepare the bio-adhesive, GDHC and GCA were dissolved at 10 wt. % in phosphate buffered saline (PBS, pH 7.4) in an incubator. The bio-adhesive precursors were then mixed with NaIO₄ at 0.1 wt.%, and 100 µL were deposited between two particular surfaces, i.e. two titanium slices ("Ti-Ti"), or between two slices of tenderloin ("tissue-tissue"), or between titanium and tissue, and pressed for about 2s on top of the other. The time it took (in seconds) to stick the two together is summarized in the table 1 below:

**Table 1.**

| | **GDHC** | **GCA** | **Ti-Ti** | **Ti-tissue** | **tissue-tissue** |
|---|---|---|---|---|---|
| C1 | 100 | 0 | 2 | >60 | - |
| C2 | 0 | 100 | 18 | >60 | - |
| 3 | 50 | 50 | 16 | 6 | 22 |
| 4 | 75 | 25 | 2 | 4 | 8 |
| 5 | 25 | 75 | 2 | 4 | 37 |

As can be seen, the bio-adhesives C1 and C2 comprising only GDHC or GCA, failed in the adhesion test. The bio-adhesives 3-5 containing GDHC and GCA achieved fast and excellent adhesion between Ti-Ti, Ti-tissue and tissue-tissue.

### General applicability

Embodiment of the invention will now be described, by way of example only, with reference to the accompanying schematic drawings.

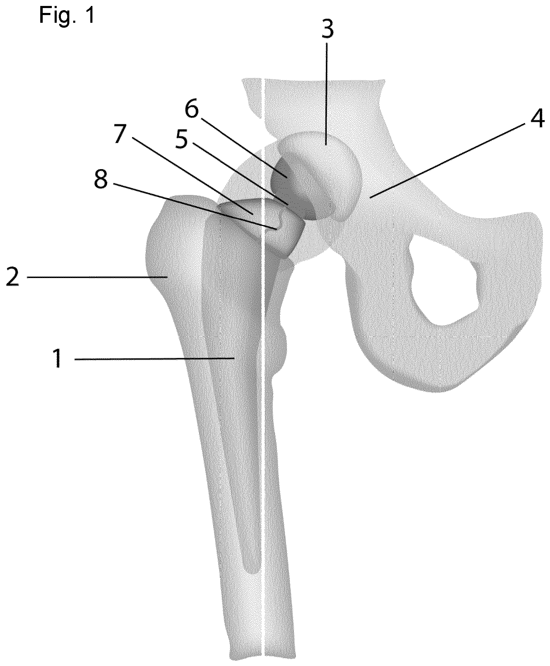

Fig. 1 shows a schematic representation of a total hip replacement. The prosthetic hip implant comprises a femoral component (1) to be connected to the femur of the patient (2) and an acetabular component (3) to be connected to the acetabulum (4) of the patient. The femoral component (1) comprises a stem to be placed into the femur, a neck (5) and a head (6) supported by the neck. Arranged on the neck (5) is a drug delivery sleeve element (7) which is fixed onto the neck (5) of the femoral component (1) using the invention from the current patent either by adhesion of a horse-shoe shape ring onto itself (8) or by adhering a sleeve shaped element directly onto the neck (5) of the femoral component (1).

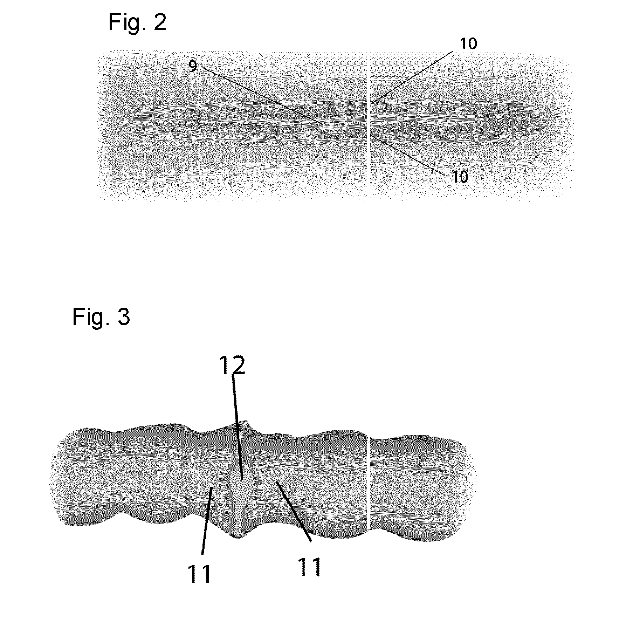

Fig. 2 shows a schematic representation of the invention (9) being used to approximate the wound margins (10) of a surgical/traumatic wound to ascertain closure of the wound without vascular compromise generally introduced by standard sutures or staples. In a similar fashion, the invention can be used to approximate other anatomical structures or organs, such as, but not limited to, muscle, fascia, tendons, subcutaneous tissue, fat or lacerations in solid organs.
Fig 3. Shows a schematic representation of the current invention being used to adhere two hollow organs together, more specifically an intestinal anastomosis, where two sides of an intestinal segment (11) are glued together using the current invention (12). The current invention may also be used to adhere other hollow structures, such as arteries, veins, lymph vessels or ducts.

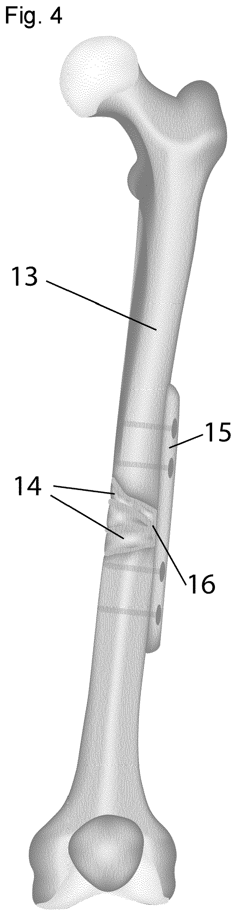

Fig. 4 shows a schematic representation of a comminuted fracture of the femur (13) with bone fragments (14) too small to be fixated by a surgical plate (15), rod, screw or any other method of instrumented fracture fixation, where the invention (16) is used to adhere these small fragments onto the larger fragments to maximize bone stock after fracture healing.

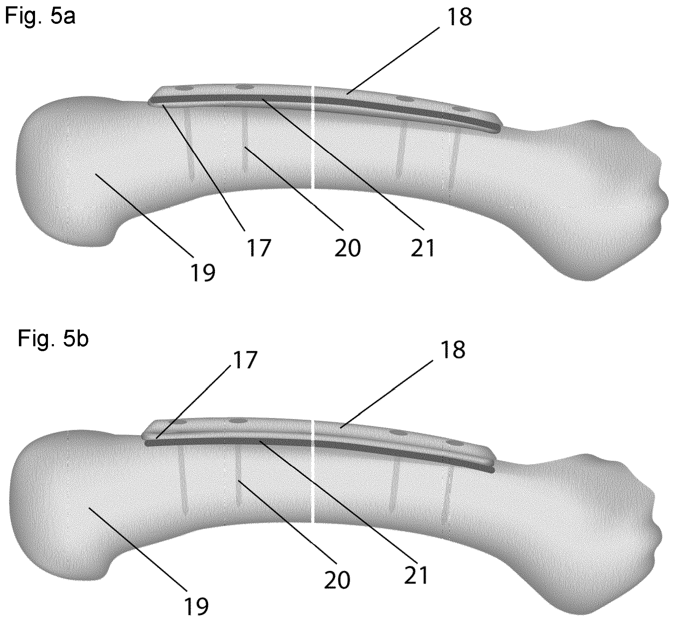

Figure 5a shows a schematic representation of a hydrogel (17) beneath a surgical plate (18) which is attached to the bone (19) via surgical screws (20), where the hydrogel (17) is adhered to the surgical plate (18) using the current invention (21). In a similar fashion, the current invention can be used to adhere hydrogels to other metallic implants.
Figure 5b shows a schematic representation of a hydrogel (17) beneath a surgical plate (18) which is attached to the bone (19) via surgical screws (20), where the hydrogel (17) is adhered to the bone (19) using the current invention (21). In a similar fashion, the current invention can be used to adhere hydrogels to other (soft) tissue.

## Claims

1. A bio-adhesive comprising one or more protein-based polymers with catechol and quinone functionality wherein the polymer is substituted with at least 3-(3,4-dihydroxyphenyl)propanoic acid (dihydrocaffeic acid, DHC) and 3-(3,4-dihydroxyphenyl)propenoic acid (caffeic acid, CA), wherein the DHC and CA are oxidized to a different degree, wherein
- the protein-based polymer is selected from silk, fibrin, collagen and/or gelatin, making up at least 50% by weight on the entire polymer content of the bio-adhesive, and
- the ratio of the DHC and CA and amount of oxidant are selected such as to achieve a conversion of 4 to 96% of the catechol moieties into quinone moieties.

2. The bio-adhesive of claim 1, wherein the protein-based polymer is gelatin.

3. The bio-adhesive according to any one of claims 1-2, wherein the protein-based polymer has a molecular weight in the range of 20 to 250 kDa, preferably in the range of 50 to 200 kDa, more preferably in the range of 90 to 150 kDa.

4. The bio-adhesive according to any one of claims 1-3, wherein the DHC and CA are at a molar ratio in the range of 10:1 to 1:10, preferably at a molar ratio in the range of 5:1 to 1:5.

5. The bio-adhesive according to any one of claims 1-4, wherein two or more protein-based polymers are used, with one protein-based polymer having a backbone onto which one functionalization agent is chemically bound and at least one protein-based polymer onto which another functionalization agent is chemically bound.

6. The bio-adhesive according to any one of claims 1-5, wherein the one or more protein-based polymers comprise the DHC and CA in an amount of 20-50% of the combined amino groups and carboxylic acid groups of the one or more protein-based polymers.

7. The bio-adhesive according to any one of claims 1-6, wherein the ratio of the DHC and CA and amount of oxidant are selected such as to achieve a conversion of 10 to 90%, preferably 20 to 80%, more preferably 30 to 70% of the catechol moieties into quinone moieties.

8. The bio-adhesive according to any one of claims 1-7, for adhering tissue, bone, metal or hydrogel onto tissue, bone, metal or hydrogel.

9. The bio-adhesive according to any one of claims 1-8, for application on the outside or inside of a living human or animal body.

10. Kit of parts comprising the bio-adhesive according to any one of claims 1-9, the kit optionally comprising a dual barrel syringe, connected to a mixing tip.

## Patentansprüche

1. Bioadhäsiv, umfassend ein oder mehrere proteinbasierte Polymere mit Catechol- und Chinonfunktionalität, wobei das Polymer mit mindestens 3-(3,4-Dihydroxyphenyl)propansäure (Dihydrokaffeinsäure, DHC) und 3-(3,4-Dihydroxyphenyl)propensäure (Kaffeinsäure, CA) substituiert ist, wobei DHC und CA in unterschiedlichem Maße oxidiert sind, wobei
- das proteinbasierte Polymer ausgewählt ist aus Seide, Fibrin, Kollagen und/oder Gelatine und mindestens 50 Gew.-% des gesamten Polymergehalts des Bioadhäsivs ausmacht, und
- das Verhältnis von DHC und CA und die Menge des Oxidationsmittels derart ausgewählt sind, um eine Umwandlung von 4 bis 96 % der Catechol-Gruppen in Chinon-Gruppen zu erreichen.

2. Bioadhäsiv nach Anspruch 1, wobei das proteinbasierte Polymer Gelatine ist.

3. Bioadhäsiv nach einem der Ansprüche 1 bis 2, wobei das proteinbasierte Polymer ein Molekulargewicht im Bereich von 20 bis 250 kDa hat, vorzugsweise im Bereich von 50 bis 200 kDa, bevorzugter im Bereich von 90 bis 150 kDa.

4. Bioadhäsiv nach einem der Ansprüche 1 bis 3, wobei DHC und CA in einem Molverhältnis im Bereich von 10:1 bis 1:10, vorzugsweise in einem Molverhältnis im Bereich von 5:1 bis 1:5, sind.

5. Bioadhäsiv nach einem der Ansprüche 1 bis 4, wobei zwei oder mehr proteinbasierte Polymere verwendet werden, mit einem proteinbasierten Polymer mit einem Grundgerüst, auf dem ein Funktionalisierungsmittel chemisch gebunden ist, und mindestens ein proteinbasiertes Polymer, auf dem ein anderes Funktionalisierungsmittel chemisch gebunden ist.

6. Bioadhäsiv nach einem der Ansprüche 1 bis 5, wobei das eine oder die mehreren proteinbasierten Polymere DHC und CA in einer Menge von 20 bis 50 % der kombinierten Aminogruppen und Carbonsäuregruppen des einen oder der mehreren proteinbasierten Polymere umfassen.

7. Bioadhäsiv nach einem der Ansprüche 1 bis 6, wobei das Verhältnis von DHC und CA und die Menge des Oxidationsmittels derart ausgewählt sind, um eine Umwandlung von 10 bis 90 %, vorzugsweise 20 bis 80 %, bevorzugter 30 bis 70 % der Catechol-Gruppen in Chinon-Gruppen zu erreichen.

8. Bioadhäsiv nach einem der Ansprüche 1 bis 7 zum Verkleben von Gewebe, Knochen, Metall oder Hydrogel auf Gewebe, Knochen, Metall oder Hydrogel.

9. Bioadhäsiv nach einem der Ansprüche 1 bis 8 zur Anwendung auf der Außen- oder Innenseite eines lebenden menschlichen oder tierischen Körpers.

10. Bausatz, umfassend das Bioadhäsiv nach einem der Ansprüche 1 bis 9, der Bausatz optional umfassend eine Doppelspritzenhülse, verbunden mit einer Mischspitze.

## Revendications

1. Bioadhésif comprenant un ou plusieurs polymères à base de protéine ayant des fonctionnalités catéchol et quinone, dans lequel le polymère est substitué par au moins l'acide 3-(3,4-dihydroxyphényl)propanoïque (acide dihydrocaféique, DHC) et l'acide 3-(3,4-dihydroxyphényl)propénoïque (acide caféique, CA), dans lequel le DHC et le CA sont oxydés à des degrés différents, dans lequel
- le polymère à base de protéine est choisi parmi la soie, la fibrine, le collagène et/ou la gélatine, constituant au moins 50 % en poids de la teneur totale en polymère du bioadhésif, et
- le rapport du DHC et du CA et la quantité d'oxydant sont choisis de façon que soit atteinte une conversion de 4 à 96 % des fragments catéchol en fragments quinone.

2. Bioadhésif selon la revendication 1, dans lequel le polymère à base de protéine est la gélatine.

3. Bioadhésif selon l'une quelconque des revendications 1 à 2, dans lequel le polymère à base de protéine a un poids moléculaire situé dans la plage allant de 20 à 250 kDa, de préférence dans la plage allant de 50 à 200 kDa, mieux encore dans la plage allant de 90 à 150 kDa.

4. Bioadhésif selon l'une quelconque des revendications 1 à 3, dans lequel le DHC et le CA sont présents en un rapport molaire situé dans la plage allant de 10/1 à 1/10, de préférence en un rapport molaire situé dans la plage allant de 5/1 à 1/5.

5. Bioadhésif selon l'une quelconque des revendications 1 à 4, dans lequel deux polymères à base de protéine ou plus sont utilisés, avec un polymère à base de protéine qui a une charpente sur laquelle un agent de fonctionnalisation est lié chimiquement et au moins un polymère à base de protéine sur lequel un autre agent de fonctionnalisation est lié chimiquement.

6. Bioadhésif selon l'une quelconque des revendications 1 à 5, dans lequel le ou les polymères à base de protéine comprennent le DHC et le CA en une quantité de 20 à 50 % des groupes amino et des groupes carboxyliques combinés du ou des polymères à base de protéine.

7. Bioadhésif selon l'une quelconque des revendications 1 à 6, dans lequel le rapport du DHC et du CA et la quantité d'oxydant sont choisis de façon que soit atteinte une conversion de 10 à 90 %, de préférence de 20 à 80 %, mieux encore de 30 à 70 % des fragments catéchol en fragments quinone.

8. Bioadhésif selon l'une quelconque des revendications 1 à 7 pour l'adhésion de tissu, os, métal ou hydrogel sur un tissu, os, métal ou hydrogel.

9. Bioadhésif selon l'une quelconque des revendications 1 à 8, pour une application sur l'extérieur ou l'intérieur d'un corps humain ou animal vivant.

10. Kit de pièces comprenant le bioadhésif selon l'une quelconque des revendications 1 à 9, le kit comprenant éventuellement une seringue double corps, connectée à une canule de mélange.
